(19) Europäisches Patentamt
European Patent Office
Office européen des brevets

(11) Veröffentlichungsnummer: **0 152 372 B1**

(12) # EUROPÄISCHE PATENTSCHRIFT

(45) Veröffentlichungstag der Patentschrift: **27.12.91**

(51) Int. Cl.5: **C07D 209/48**, C08G 73/12, C08F 26/06, C07D 209/76, C08F 22/40

(21) Anmeldenummer: **85810024.1**

(22) Anmeldetag: **25.01.85**

(54) **Substituierte ungesättigte bicyclische Imide und deren Polymeren.**

(30) Priorität: **31.01.84 CH 450/84**
**05.10.84 CH 4796/84**

(43) Veröffentlichungstag der Anmeldung:
**21.08.85 Patentblatt 85/34**

(45) Bekanntmachung des Hinweises auf die Patenterteilung:
**27.12.91 Patentblatt 91/52**

(84) Benannte Vertragsstaaten:
**CH DE FR GB IT LI NL**

(56) Entgegenhaltungen:
**EP-A- 0 105 024**
**EP-A- 0 146 498**

(73) Patentinhaber: **CIBA-GEIGY AG**
**Klybeckstrasse 141**
**CH-4002 Basel(CH)**

(72) Erfinder: **Renner, Alfred, Dr.**
**Marcoup 2**
**CH-3280 Muntelier(CH)**

EP 0 152 372 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft mit Allyl oder Methallyl substituierte Methyl-bicyclo[2.2.1]hept-5-en-2,3-dicarbonsäureimide, deren Herstellung und die daraus durch Erwärmen erhältlichen Polymeren.

Maleinimide und Bismaleinimide sowie N-Allyl-monomaleinimide sind bekannt.

Im US Patent 3 334 075 wird die Härtung von halogenierten olefinischen Gummipolymeren mit ausgewählten Polymaleinimidverbindungen, wie N,N'-m-Phenylen-bismaleinimid, beschrieben. Diese Polymaleinimide enthalten keine Allyl- oder Norbornenylgruppen, und die Maleinimidreste tragen keine Substituenten.

Aus dem britischen Patent 1 277 790 sind harzbildende Zusammensetzungen bekannt, welche Maleinimid- oder Bismaleinimidderivate, wie N-Phenyl-maleinimid und Methylen-bis-(N-phenyl-maleinimid), enthalten. Keine dieser Verbindungen enthält Norbornenyl- oder Allylgruppen.

Im US Patent 3 839 358 wird ein Verfahren zur Herstellung von Bismaleinimiden durch Umsetzung einer Bismaleinamidsäure mit dem Anhydrid einer niederen Carbonsäure in Gegenwart eines tertiären Amins, eines organischen Lösungsmittels und eines Nickelkatalysators beschrieben. Aus dem US Patent 4 229 351 ist ein Verfahren zur Herstellung von am Stickstoffatom aliphatisch substituierten Mono- und Bismaleinimiden bekannt. Weder im einen noch im anderen Patent wird die Herstellung von Verbindungen mit allyl- und methylsubstituierten Norbornenylgruppen beschrieben oder nahegelegt.

Das US Patent 3 450 711 betrifft Bisimidverbindungen, hergestellt durch Umsetzung von Endo,cis-bicyclo[2.2.1]hept-5-en-2,3-dicarbonsäureanhydrid (= 5-Norbornen-2,3-dicarbonsäureanhydrid) mit ausgewählten organischen Diaminen. Diese Bisimide enthalten weder Methyl- noch Allylsubstituenten im Imidrest und unterscheiden sich von den vorliegenden Verbindungen sowohl durch ihre Struktur als auch durch ihre chemische Reaktivität. Die Verbindungen gemäss diesem US Patent werden als Zwischenprodukte bei der Herstellung von Epoxidverbindungen verwendet.

Ferner ist bekannt, dass durch Addition von 3,3',4,4'-Benzophenontetracarbonsäuredianhydrid und Diaminodiphenylmethan in Gegenwart verschiedener zur Vernetzung und zur Endgruppenverkappung befähigter Verbindungen, wie gegebenenfalls chloriertes 5-Norbornencarbonsäureanhydrid, und 5-Vinylphthalsäureanhydrid, Polyimid-Oligomere hergestellt werden können, die als Klebstoffe Anwendung finden [vgl. z.B. Polym. Eng.Sci., 22, 9-14 (1982)]. Diese Polyimid-Oligomeren enthalten keine Allylgruppen.

Im US Patent 4 271 074 werden Silane, hergestellt aus Imidzwischenprodukten, z.B. auch N-Allyl-2,3-dimethyl-maleinimid, beschrieben. Die erfindungsgemässen Monomeren weisen keine Norbornenylgruppe, welche mit einer Allyl- und einer Methylgruppe substituiert ist, auf, sind daher strukturell ganz verschieden und durch dieses Patent nicht nahegelegt.

Die Herstellung der Ausgangsprodukte für die erfindungsgemässen Verbindungen wird im US Patent 3 105 839 beschrieben.

Die erfindungsgemässen allyl- oder methallylsubstituierten Methylbicyclo[2.2.1]hept-5-en-2,3-dicarbonsäureimide sind wertvolle Ausgangsprodukte für Polymere mit ausgezeichneten Eigenschaften. Sie sind durch die folgende Formel I gekennzeichnet:

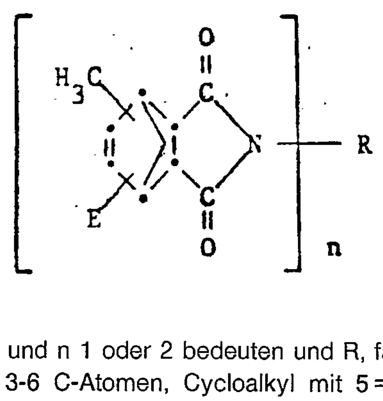 (I),

worin E Allyl oder Methallyl und n 1 oder 2 bedeuten und R, falls n 1 bedeutet, für Wasserstoff, Alkyl mit 1-12 C-Atomen, Alkenyl mit 3-6 C-Atomen, Cycloalkyl mit 5=8 C-Atomen, Aryl mit 6-10 C-Atomen oder Benzyl oder, falls n 2 bedeutet, für $-C_mH_{2m}-$ mit m = 2-20, Arylen mit 6-10 C-Atomen oder für eine Gruppe der Formel II

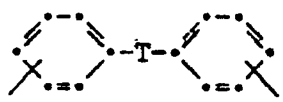 (II) ;

worin T Methylen, Isopropyliden, CO, O, S oder $SO_2$ bedeutet, steht.

Vorzugsweise bedeutet E die Allylgruppe.

R kann eine gerad- oder verzweigtkettige Alkylgruppe mit 1-12 C-Atomen bedeuten, wie Methyl, Aethyl, Isopropyl, n-Butyl, Isopentyl, n-Hexyl, 2-Aethyl-hexyl, n-Decyl und n-Dodecyl, vorzugsweise Alkyl mit 1-8 C-Atomen.

R kann auch eine geradkettige oder verzweigtkettige Alkenylgruppe mit 3-6 C-Atomen bedeuten, wie Allyl, Methallyl, 2-Butenyl und 3-Hexenyl, vorzugsweise Allyl.

Wenn R eine Cycloalkylgruppe bedeutet, kann es sich um eine Cyclopentyl-, Cyclohexyl-, Cycloheptyl- oder Cyclooctylgruppe handeln, vorzugsweise um Cyclohexyl.

R in der Bedeutung einer Arylgruppe kann unsubstituiertes Phenyl oder eine durch eine oder zwei Methylgruppen substituierte Phenylgruppe sein, wie Tolyl oder Xylyl, oder auch Naphthyl. Bevorzugt ist die Phenylgruppe. Stellt R eine Gruppe $-C_mH_{2m}-$ dar, so kann es sich um geradkettige oder verzweigte Reste handeln, wie Aethylen, Propylen, Trimethylen, Tetramethylen, Hexamethylen, Octamethylen und Dodecamethylen. Bedeutet R eine Gruppe der Formel II, so ist diese vorzugsweise in 4,4'-Stellung an die N-Atome gebunden.

Vorzugsweise bedeutet R eine Gruppe $-(CH_2)_m-$ mit m = 2 bis 12.

R kann in der Bedeutung einer Arylengruppe mit 6-10 C-Atomen z.B. eine m-Phenylen-, p-Phenylen-, 1,3-Naphthylen-, 1,4-Naphthylen-1,5-Naphthylen- oder 2,6-Naphthylengruppe bedeuten.

Wenn R eine Gruppe der Formel II bedeutet, steht T vorzugsweise für die Methylengruppe, O oder $SO_2$.

Bevorzugte Verbindungen der Formel I sind solche, worin R, falls n = 1 ist, für Wasserstoff, Alkyl mit 1-8 C-Atomen, Cyclohexyl, Allyl oder Phenyl steht, oder, falls n = 2 ist, für $-(CH_2)_6-$ oder für eine Gruppe der Formel II steht, worin T die Methylengruppe oder $SO_2$ bedeutet.

Besonders bevorzugt sind Verbindungen der Formel I, worin
n die Zahl 2 und R $-(CH_2)_6-$,

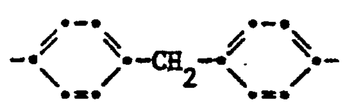

oder

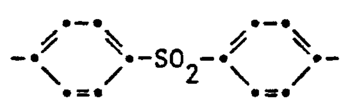

bedeuten.

Ganz besonders bevorzugt sind Verbindungen der Formel I, worin E die Allylgruppe bedeutet und R, falls n = 1, für Allyl steht,
oder, falls n = 2, für $-(CH_2)_6-$ oder für

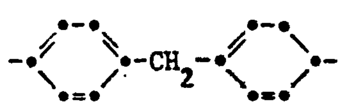

steht.

Die erfindungsgemässen Imide können auf an sich bekannte Weise z.B. durch Umsetzen eines Anhydrids der Formel III

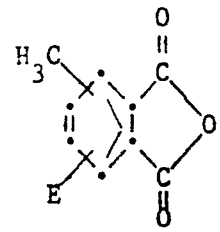 (III)

mit einer Verbindung der Formel IV

$(H_2N)_n-R$ (IV) ,

worin E, R und n die unter Formel I angegebene Bedeutung haben, bei erhöhter Temperatur und unter Abdestillieren des bei der Reaktion entstehenden Wassers hergestellt werden. Sofern es sich bei den Verbindungen der Formel IV um Ammoniak oder niedrigsiedende Monoamine handelt, ist ein Ueberschuss dieser Reaktanden zu empfehlen. Diamine sind vorteilhaft in stöchiometrischem Verhältnis einzusetzen. Die Umstetzung kann ohne Lösungsmittel oder in Gegenwart eines inerten Lösungsmittels, das für die azeotrope Entfernung des Wassers verwendbar ist (Schleppmittel) erfolgen. Die Temperatur der Umsetzung kann zwischen 100 und 250°C liegen. Bevorzugt werden die Imide der Formell I in der Schmelze bei einem Druck von höchstens 4500 Pa bei Temperaturen zwischen 130 und 220°C, insbesondere 180 und 220°C,hergestellt.

Wie schon erwähnt, können die Ausgangsstoffe der Formel III gemäss dem in der US Patentschrift 3 105 639 beschriebenen Verfahren hergestellt werden, indem man Natrium-Methylcyclopentadienid mit einem Allyl- oder Methallylhalogenid umsetzt, worauf sich eine Diels-Alder-Reaktion mit Maleinsäureanhydrid anschliesst. Obgleich in der US Patentschrift angegeben wird, dass die Allylgruppe in 7-Stellung des bicyclischen Systems gebunden ist, zeigen neuere Untersuchungen, dass eine isomere Mischung in Bezug auf die Stellung der Allylgruppe (in 1 und 6 Position) und auch auf die Endo- und Exokonfiguration des Anhydridteils gebildet wird. Die isomeren Komponenten konnten bisher nur durch präparative Gaschromatographie isoliert werden.

Die verwendeten Monoamine oder Diamine der Formel IV sind bekannt oder können nach an sich bekannten Verfahren hergestellt werden.

Die erfindungsgemässen Verbindungen sind flüssige oder niedrigschmelzende feste Stoffe, die zu festen Produkten mit hoher Glasumwandlungstemperatur und Wärme- und Wasserbeständigkeit polymerisiert werden können. Diese Produkte können vielseitig angewendet werden, z.B. als Giessharze oder Klebstoffe und insbesondere zur Herstellung von glasfaser- oder kohlenstoffaserverstärkten Kunststoffen, hitzebeständigen Verbundwerkstoffen, sowie als elektrische Isolierstoffe und Wirbelsinterpulverlacke.

Die erfindungsgemässen Verbindungen können direkt verwendet und polymerisiert werden, oder sie können zunächst in einem organischen Lösungsmittel, wie Toluol, Xylol, Methyläthylketon, Aethylenglykolmonoalkyl- und -dialkyläthern mit 1-4 C-Atomen in den Alkylgruppen oder einem ähnlichen, in der Lackindustrie üblichen Lösungsmittel, gelöst werden. Solche Lösungen können als Imprägniermittel oder Beschichtungsmittel oder auch als Versandobjekt an den Verbraucher dienen.

Die erfindungsgemässen Verbindungen der Formel I können zu neuen Polymeren umgesetzt werden, wobei überraschenderweise der Methylsubstituent die Polymerisationsfähigkeit des Allylnorbornensystems kaum beeinträchtigt. Gegenstand der Erfindung sind demnach auch die neuen Polymeren, die dadurch erhältlich sind, dass man ein Imid der Formel I während 6 bis 60 Stunden auf eine Temperatur zwischen 180 und 300°C, vorzugsweise zwischen 200 und 250°C erhitzt. Dabei gilt in Bezug auf bevorzugte Bedeutungen von E, R und n das oben Angegebene. Besonders bevorzugt sind Polymere, die dadurch erhältlich sind, dass man ein Imid der Formel I, worin n die Zahl 2 und R die Gruppe

$-(CH_2)_6-$, 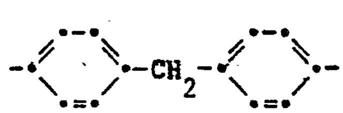 oder 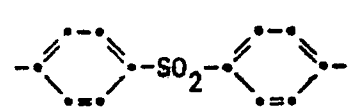

bedeuten, während 6-24 Stunden auf 240 bis 250°C erhitzt.

Selbstverständlich können den Imiden der Formel I inerte und stabile Stoffe, wie Füllmittel, Pigmente, Farbstoffe und andere Additive, zugegeben werden, bevor sie zu vernetzten Gebilden polymerisiert werden.

HERSTELLUNGSBEISPIELE

Beispiel 1: Allyl-methyl-bicyclo[2.2.1]hept-5-en-2,3-dicarbonsäureimid

Man erhitzt eine Mischung aus 30 g Allyl-methyl-bicyclo[2.2.1]hept-5-en-2,3-dicarbonsäureanhydrid, hergestellt gemäss Beispiel 2 der US Patentschrift 3 105 839, und 10,2 g 25%iger wässriger Ammoniaklösung unter Rühren und Rückflusskühlung während 2,5 Stunden auf 100-108 °C. Gemäss Gaschromatogramm erhält man ein Gemisch von 12 Isomeren mit variablen Positionen der Allyl- und Methylgruppe in den 1-, 4-, 5-und 6-Stellungen des Bicycloheptenrings sowie Exo- und Endo-anhydride. Da dieses Isomerengemisch durch fraktionierte Destillation nicht zu trennen ist, wird es als solches weiter verwendet. Danach destilliert man Wasser ab, treibt überschüssigen Ammoniak aus und rektifiziert das Imid bei 140-145 °C und 2,0 Pa.

Man erhält 21,5 g eines Isomerengemisches von Allyl-methyl-bicyclo-[2.2.1]hept-5-en-dicarbonsäure-2,3-imid, was einer Ausbeute von 71,3 % d.Th. entspricht. Das Imid ist ein gelber Syrup und mit einer Viskosität von 96,18 mPa.s bei 80 °C.

| Analyse | % C | % H | % N |
|---|---|---|---|
| berechnet für $C_{13}H_{15}NO_2$ | 71,87 | 6,96 | 6,45 |
| gefunden | 71,41 | 6,94 | 6,42 |

| IR-Spektrum: | |
|---|---|
| 1639,2 $cm^{-1}$ | cyclische Doppelbindung |
| 1653,4 $cm^{-1}$ | Allylgruppe |
| 1708,9 $cm^{-1}$ | Carbonylgruppe |
| 1778,0 $cm^{-1}$ | Carbonyl im cyclischen Imid |
| 3210,3 $cm^{-1}$ | NH-Schwingung |

Nach der Polymerisation während 48 Stunden bei 250 °C erhält man einen Festkörper mit einer Glasumwandlungstemperatur (GUT) von 125 °C. Im IR-Spektrum finden sich keine Absorptionen für

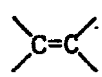

Doppelbindungen (1639,2 und 1653,4 $cm^{-1}$).

Beispiel 2: Allyl-methyl-bicyclo[2.2.1]hept-5-en-2,3-dicarbonsäure-N-allylimid.

Man erhitzt eine Mischung aus 30 g Allyl-methyl-bicyclo]2.2.1]hept-5-en-2,3-dicarbonsäureanhydrid und 9,41 g Allylamin während 2 Stunden auf Rückfluss, destilliert Wasser ab und rektifiziert das Produkt bei 119-127 °C und 2,66 Pa. Man erhält 30,24 g (85,5 % der Theorie) eines hellgelben Oels mit folgenden Kenndaten: $n_D^{20}$ = 1,5202, $\eta_{25}$ =0,135 Pa.s.

| Analyse: | % C | % H | % N |
|---|---|---|---|
| berechnet für $C_{16}H_{19}NO_2$ | 74,68 | 7,84 | 5,44 |
| gefunden | 74,66 | 7,55 | 5,22 |

Die Polymerisation während 48 Stunden bei 250 °C ergibt einen Festkörper mit einer GUT >250 °C, dessen IR-Spektrum keine

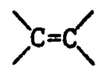

Absorptionsfrequenzen (1639,2 und 1653,4 $cm^{-1}$) aufweist.

Beispiel 3: Allyl-methyl-bicyclo[2.2.1]hept-5-en-2,3-dicarbonsäure-N-(2-äthylhexyl)imid.

Wie im vorhergehenden Beispiel 2 beschrieben, setzt man 30 g Anhydrid mit 21,3 g 2-Aethylhexylamin um. Bei der Destillation bei 3,3 Pa erhält man zwischen 149 und 162 °C 40,74 g eines gelben Oels (90 % der Theorie) mit einer Brechungszahl $n_D^{20}$ = 1,5090 und einer Viskosität von 0,306 Pa.s bei 25 °C.

| Analyse | % C | % H | % N |
|---|---|---|---|
| berechnet für $C_{21}H_{31}NO_2$<br>gefunden | 76,55<br>76,32 | 9,48<br>9,48 | 4,52<br>4,16 |

Nach der Polymerisation während 40 Stunden bei 250 °C erhält man einen Festkörper mit einer GUT von 128 °C. Die Doppelbindungs-IR-Banden bei 1639,2 und 1653,4 $cm^{-1}$ sind nicht mehr nachweisbar.

Beispiel 4: Allyl-methyl-bicyclo[2.2.1]hept-5-en-2,3-dicarbonsäure-N-phenylimid.

Wie im Beispiel 2 beschrieben, setzt man 30 g Anhydrid mit 15,4 g Anilin um. Man destilliert das Anilid zwischen 161 und 165 °C bei 6,65 Pa und erhält 23,2 g (57,5 % d.Th.) einer zähen Flüssigkeit mit einer Viskosität von 4,2 Pa.s bei 40 °C und einer Brechungszahl $n_D^{20}$ = 1,5647 bei 20 °C.

| Analyse | % C | % H | % N |
|---|---|---|---|
| berechnet für $C_{19}H_{19}NO_2$<br>gefunden | 77,79<br>77,92 | 6,53<br>6,46 | 4,77<br>4,68 |

Die Polymerisation während 48 Stunden bei 250 °C ergibt einen Festkörper mit einer GUT von 132,5 °C dessen IR-Spektrum keine

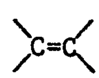

Absorptionsfrequenzen (1639,2 und 1653,4 $cm^{-1}$) aufweist.

Beispiel 5: N,N'-Hexamethylen-bis-(allyl-methyl-bicyclo[2.2.1]hept-5-en-2,3-dicarbonsäureimid)

Man legt 300 g Allyl-methyl-bicyclo[2.2.1]hept-5-en-2,3-dicarbonsäureanhydrid vor, erhitzt auf 130 °C und tropft unter Rühren 79,9 g geschmolzenes Hexamethylendiamin ein. Man destilliert Wasser ab, steigert die Temperatur auf 180 °C, und erniedrigt den Druck auf 53 Pa. Man hält 15 min auf 180 °C und 53 Pa. Man erhält 345 g eines braunen, zähen Harzes (97 % d.Th.) mit einer Viskosität von 1,356 Pa.s bei 80 °C.

| Analyse | % C | % H | % N |
|---|---|---|---|
| berechnet für $C_{32}H_{40}N_2O_4$<br>gefunden | 74,39<br>73,82 | 7,80<br>7,74 | 5,42<br>5,47 |

Beispiel 6: Bis[4-(allyl-methyl-bicyclo[2.2.1]hept-5-en-2,3-dicarbonsäureimidophenyl)]methan

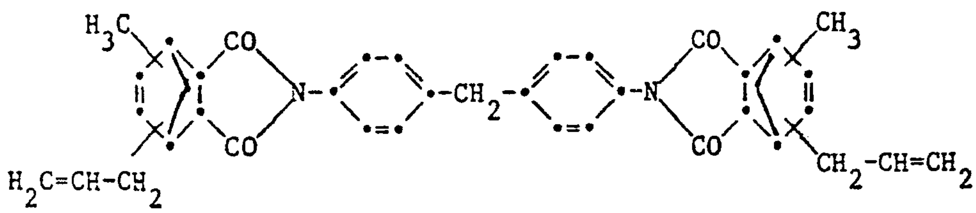

Man setzt 300 g Anhydrid und 136,2 g 4,4'-Diaminodiphenylmethan so um, wie dies im Beispiel 5 beschrieben ist. Man erhält 403,2 g (98,0 % d.Th.) eines dunkelbraunen Festharzes mit einer Glasumwandlungstemperatur von 66 °C.

| Analyse: | % C | % H | % N |
|---|---|---|---|
| berechnet für $C_{39}H_{38}N_2O_4$: | 78,24 | 6,40 | 4,68 |
| gefunden: | 78,41 | 6,49 | 4,68 |

Beispiel 7: Bis[4-(methallyl-methyl-bicyclo[2.2.1]hept-5-en-2,3-dicarbonsäureimidophenyl)]sulfon

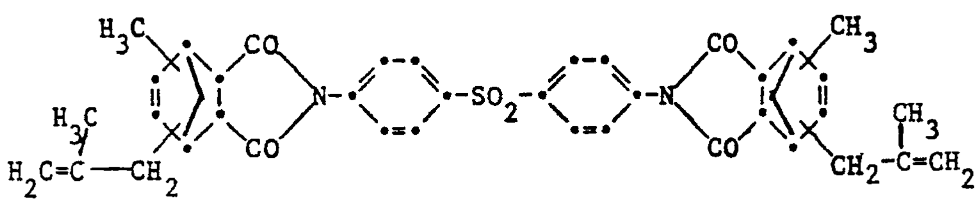

Man erhitzt 124,15 g 4,4'-Diaminodiphenylsulfon und 232 g Methallyl-methylbicyclo[2.2.1]hept-5-en-2,3-dicarbonsäureanhydrid auf 180 °C und erniedrigt den Druck sukzessive auf 25 Pa. Nach 90 min bei 180 °C und 25 Pa erhält man 319,55 g eines braunen Festharzes mit einer Glasumwandlungstemperatur von 87 °C.

| Analyse: | % C | % H | % N | % S |
|---|---|---|---|---|
| berechnet für $C_{40}H_{40}N_2O_6S$: | 70,98 | 5,96 | 4,14 | 4,74 |
| gefunden: | 69,98 | 5,91 | 4,35 | 4,98 |

Das Anhydrid wird analog den Beispielen 1 und 2 der US Patentschrift 3 105 839 hergestellt. Anstelle von 840 g Allylchlorid verwendet man 994 g Methallylchlorid. Das bisher in der Literatur nicht beschriebene Anhydrid destilliert bei 125 - 140 °C, 25 Pa und hat ein $n_D^{20}$ = 1,508 und eine Viskosität von 195 mPa.s.

Beispiel 8: Bis[4-(methallyl-methyl-bicyclo[2.2.1]hept-5-en-2,3-dicarbonsäureimidophenyl)]methan

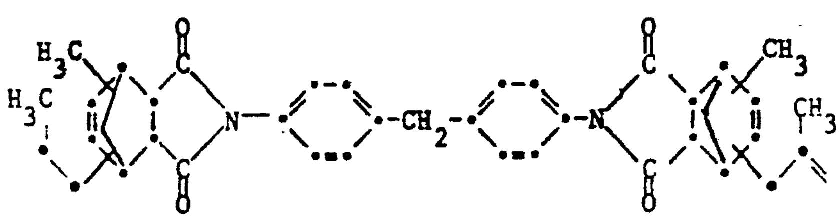

Man erhitzt 116 g Methallyl-methyl-bicyclo [2.2.1]hept-5-en-2,3-dicarbonsäureanhydrid und 49,5 g 4,4'-Diaminodiphenylmethan unter Rühren in einer $N_2$-Atmosphäre auf 200 °C. Es destillieren 9 $cm^3$ Wasser ab. Im Verlauf von 35 min bei 200 °C steigt die Glasumwandlungstemperatur von 67,5 auf 78,5 °C. Ausbeute 155 g (99 % d. Th.).

| Analyse: | %C | %H | %N |
|---|---|---|---|
| berechnet für $C_{41}H_{42}N_2O_4$: | 78,57 | 6,75 | 4,47 |
| gefunden: | 77,41 | 6,71 | 4,39 |

Beispiel 9: Allyl-methyl-bicyclo[2.2.1]hept-5-en-2,3-dicarbonsäure-N-cyclohexylimid.

Man erhitzt eine Mischung aus 20 g Allyl-methyl-bicyclo[2.2.1]hept-5-en-2,3-dicarbonsäureanhydrid und 9,09 g Cyclohexylamin während 3 Stunden auf 135 °C, und destilliert danach das Produkt im Vakuum. Zwischen 128 und 138 °C destillieren bei 2,5 Pa 16 g eines gelben, viskosen Oels über, was einer Ausbeute von 58,2 % entspricht.

| Analyse: | %C | %H | %N |
|---|---|---|---|
| berechnet für $C_{19}H_{25}NO_2$: | 76,25 | 8,36 | 4,68 |
| gefunden: | 76,46 | 8,52 | 4,38 |

Nach der thermischen Polymerisation während 48h bei 250 °C erhält man ein Festharz mit einer Glasumwandlungstemperatur von 72,5 °C.

Die erfindungsgemässen Verbindungen haben zwei oder mehr olefinische Doppelbindungen im Molekül, die sie zur Polymerisation befähigen. Beim Erhitzen erhält man vernetzte Polymere mit wertvollen physikalischen Eigenschaften, vgl. Beispiele 1-4.

## ANWENDUNGSBEISPIELE

### Beispiel I

Das gemäss Beispiel 5 hergestellte Imid giesst man als heisse, dünnflüssige Schmelze in eine Stahlform von 12 x 12 x 0,4 cm und härtet 3 h bei 200 °, 3 h bei 225 ° und 12 h bei 250 °C aus. Nach dem Abkühlen schneidet man Prüfstäbe aus der Platte. Folgende Eigenschaften werden daran gemessen:

| | |
|---|---|
| Biegefestigkeit nach DIN 53452 : | 81,5 $N/mm^2$ |
| Durchbiegung nach DIN 53452: | 5,2 mm |
| Schlagbiegefestigkeit nach DIN 53455: | 13,2 $kJ/m^2$ |
| Glasumwandlungstemperatur (TA 2000 der Fa. Mettler) | 201 °C |
| Wasseraufnahme ($1^h$ 100°C) | 0,48 % |
| Zugscherfestigkeit auf Anticorodal nach DIN 53283 | 9,9 $N/mm^2$ |

### Beispiel II

Das gemäss Bespiel 6 hergestellte Imidharz wird aufgeschmolzen, in eine Stahlform von 12 x 12 x 0,4 cm gegossen und während 3 Stunden bei 200 °, 3 Stunden bei 225 ° und 12 Stunden bei 250 °C gehärtet. Nach dem Abkühlen zerschneidet man die Platte zu Prüfstäben, an denen folgende Eigenschaften

gemessen werden:

| | |
|---|---|
| Biegefestigkeit nach DIN 53452: | 95 $N/mm^2$ |
| Durchbiegung nach DIN 53452 | 4,5 mm |
| Schlagbiegefestigkeit nach DIN 53455: | 9,5 $kJ/m^2$ |
| Glasumwandlungstemperatur (TA 2000 der Fa. Mettler) | 192 °C |
| Wasseraufnahme ($1^h$ 100°C): | 0,35 % |
| Zugscherfestigkeit auf Anticorodal nach DIN 53283: | 7,6 $N/mm^2$ |

An einer Prüfplatte (12 x 12 x 0,2 cm) werden gemessen:

| | |
|---|---|
| Spezifischer Durchgangswiderstand (DIN 53482) | $5{,}0 \times 10^{16}$ Ωcm |
| Dielektrischer Verlustfaktor (DIN 53483) | 0,25 % |
| Dielektrizitätskonstante: (DIN 53483) | 3,2 |

Beispiel III:

Das gemäss Beispiel 7 hergestellte Harz ergibt nach 24-stündiger Härtung bei 250° C eine Glasumwandlungstemperatur >250° C und eine Zugscherfestigkeit auf Anticorodal gemäss DIN 53283 von 9,5 $N/mm^2$.

## Patentansprüche

**1.** Imide der Formel I

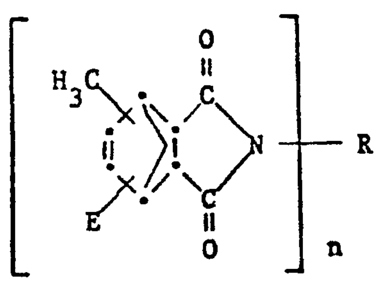 (I),

worin E Allyl oder Methallyl und n 1 oder 2 bedeuten und R, falls n 1 bedeutet, für Wasserstoff, Alkyl mit 1-12 C-Atomen, Alkenyl mit 3-6 C-Atomen, Cycloalkyl mit 5-8 C-Atomen, Aryl mit 6-10 C-Atomen oder Benzyl oder, falls n 2 bedeutet, für $-C_mH_{2m}-$ mit m = 2-20, Arylen mit 6-10 C-Atomen oder für eine Gruppe der Formel II

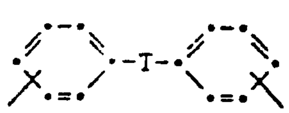 (II) ,

worin T Methylen, Isopropyliden, CO, O, S oder $SO_2$ bedeutet, steht.

**2.** Imide der Formel I gemäss Anspruch 1, worin E die Allylgruppe bedeutet.

3. Imide der Formel I gemäss Anspruch 1, worin R, falls n = 1 ist, für Wasserstoff, Alkyl mit 1-8 C-Atomen, Cyclohexyl, Allyl, oder Phenyl steht, oder, falls n = 2, für $-(CH_2)_6-$ oder für eine Gruppe der Formel II

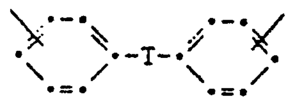

(II)

steht, worin T die Methylengruppe oder $SO_2$ bedeutet.

4. Imide der Formel I gemäss Anspruch 1, worin n die Zahl 2 und R $-(CH_2)_6-$,

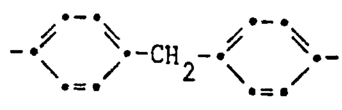

oder

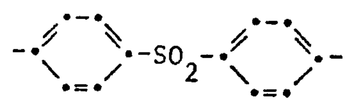

bedeuten.

5. Imide der Formel I gemäss Anspruch 1, worin E die Allylgruppe bedeutet und R, falls n = 1, für Allyl steht, oder, falls n = 2, für $-(CH_2)_6-$ oder für

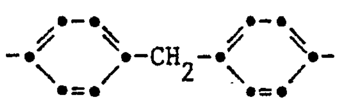

steht.

6. Verfahren zur Herstellung von Imiden der Formel I nach Anspruch 1, dadurch gekennzeichnet, dass man ein Anhydrid der Formel III

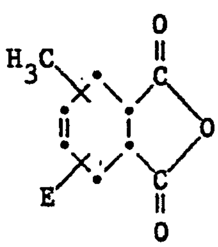

(III)

bei erhöhter Temperatur und unter Abdestillieren des bei der Reaktion entstehenden Wassers mit einer Verbindung der Formel IV

$$(H_2N)_{\overline{n}}R \qquad (IV)$$

umsetzt, wobei E, R und n die im Anspruch 1 angegebene Bedeutung haben.

7. Polymere, die dadurch erhältlich sind, dass man ein Imid der Formel I gemäss Anspruch 1 während 6 bis 60 Stunden auf eine Temperatur zwischen 180 und 300° C erhitzt.

8. Polymere nach Anspruch 7, dadurch gekennzeichnet, dass man eine Verbindung der Formel I, worin n

die Zahl 2 und R $-(CH_2)_6-$,

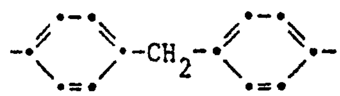 oder 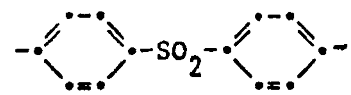

bedeuten, während 6 bis 24 Stunden auf 200 bis 250 °C erhitzt.

## Claims

**1.** An imide of the formula I

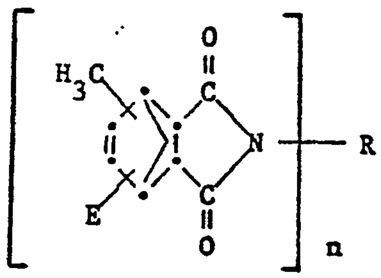 (I)

in which E is allyl or methallyl and n is 1 or 2 and, if n is 1, R is hydrogen, alkyl having 1-12 C atoms, alkenyl having 3-6 C atoms, cycloalkyl having 5-8 C atoms, aryl having 6-10 C atoms or benzyl, or, if n is 2, R is $-C_mH_{2m}-$ in which m is 2-20, arylene having 6-10 C atoms or a group of the formula II

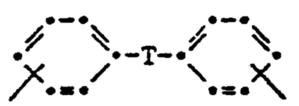 (II)

in which T is methylene, isopropylidene, CO, O, S or $SO_2$.

**2.** An imide of the formula I, according to claim 1, in which E is the allyl group.

**3.** An imide of the formula I, according to claim 1, in which, if n is 1, R is hydrogen, alkyl having 1-8 C atoms, cyclohexyl, allyl or phenyl, or, if n is 2, R is $-(CH_2)_6-$ or a group of the formula II

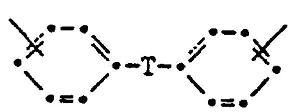 (II)

in which T is the methylene group or $SO_2$.

**4.** An imide of the formula I, according to claim 1, in which n is the number 2 and R is $-(CH_2)_6-$,

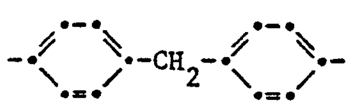 or 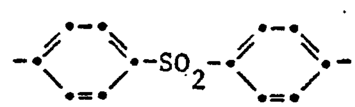

**5.** An imide of the formula I, according to claim 1, in which E is the allyl group and, if n is 1, R is allyl or,

if n is 2, R is $-(CH_2)_6-$ or

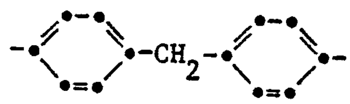

6. A process for the preparation of an imide of the formula I according to claim 1, which comprises reacting an anhydride of the formula III

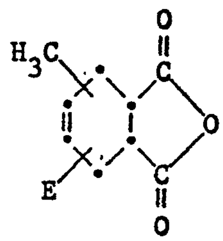

(III)

with a compound of the formula IV

$$(H_2N)_n-R$$

at an elevated temperature and with removal by distillation of the water formed in the reaction, E, R and n being as defined in claim 1.

7. A polymer obtainable by heating an imide of the formula I according to claim 1 at a temperature between 180 and 300 °C for 6 to 60 hours.

8. A polymer according to claim 7, wherein a compound of the formula I in which n is the number 2 and R is $-(CH_2)_6-$,

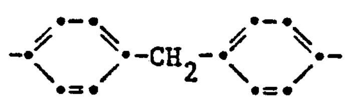

or

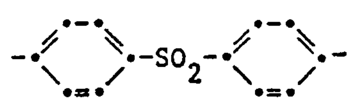

is heated at 200 to 250 °C for 6 to 24 hours.

## Revendications

1. Imides de formule I ci-dessous

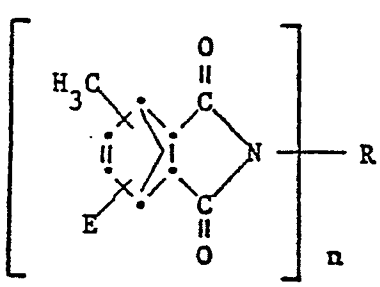

(I),

dans laquelle E représente le groupe allyle ou méthallyle, n = 1 ou 2 et R, si n = 1, désigne

l'hydrogène, un alkyle en $C_{1-12}$, un alcényle en $C_{3-6}$, un cycloalkyle en $C_{5-8}$, un aryle en $C_{6-10}$ ou un benzyle, ou bien si n = 2, un groupe $-C_mH_{2m}-$, m étant un nombre de 2 à 20, un arylène en $C_{6-10}$ ou un groupe de formule II

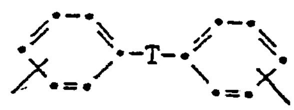

(II) ,

T représentant le groupe méthylène ou isopropylidène ou bien CO, O, S ou $SO_2$.

**2.** Imides de formule I selon la revendication 1, dans lesquels E est le groupe allyle.

**3.** Imides de formule I selon la revendication 1, dans lesquels R, si n = 1, est l'hydrogène, un alkyle en $C_{1-8}$,un cyclohexyle, un allyle ou un phényle, ou bien, si n = 2, le groupe $-(CH_2)_6-$ ou un groupe de formule II

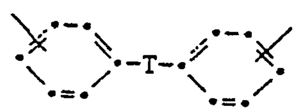

(II)

dans lequel T désigne le groupe méthylène ou $SO_2$.

**4.** Imides de formule I selon la revendication 1, dans lesquels n est le nombre 2 et R le groupe $-(CH_2)_6-$,

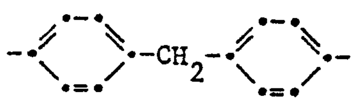

ou

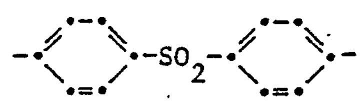

**5.** Imides de formule I selon la revendication 1, dans lesquels E est le groupe allyle et R, si n = 1, également le groupe allyle, ou bien, si n = 2, le groupe $-(CH_2)_6-$ ou

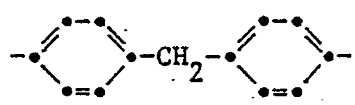

**6.** Procédé de préparation d'imides de formule I selon la revendication 1, procédé caractérisé en ce que l'on fait réagir un anhydride de formule III

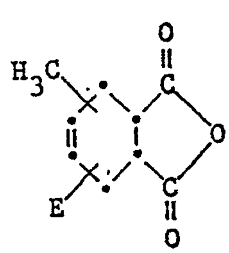

(III)

avec un composé de formule IV

$(H_2N)_n R$ (IV)

à chaud et tout en éliminant par distillation l'eau formée par la réaction.

7. Matières polymères que l'on obtient en chauffant un imide de formule I selon la revendication 1 pendant 6 à 60 heures à une température de 180 à 380 °C.

8. Matières polymères selon la revendication 7, caractérisées en ce qu'on les obtient en chauffant pendant 6 à 24 heures entre 200 et 250 °C un composé de formule I dans lequel n est le nombre 2 et R le groupe $-(CH_2)_6-$,

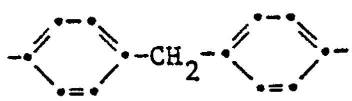

ou